# EUROPÄISCHE PATENTSCHRIFT

(11) **EP 1 976 463 B1**
(45) Veröffentlichungstag und Bekanntmachung des Hinweises auf die Patenterteilung: **01.06.2016**
(21) Anmeldenummer: 07702340.6
(22) Anmeldetag: 16.01.2007
(51) Int. Cl.: A61F 2/66, A61F 2/68

(54) **KÜNSTLICHER FUSS UND VERFAHREN ZUR STEUERUNG SEINER BEWEGUNG**
ARTIFICIAL FOOT AND METHOD FOR CONTROLLING THE MOVEMENT THEREOF
PIED ARTIFICIEL ET PROCEDE POUR COMMANDER SON MOUVEMENT

(30) Priorität: 27.01.2006 DE 102006004132
(43) Veröffentlichungstag der Anmeldung: 08.10.2008
(73) Patentinhaber: Otto Bock HealthCare GmbH, 37115 Duderstadt (DE)
(72) Erfinder: MOSLER, Lüder, 37115 Duderstadt (DE); PUSCH, Martin, 37115 Duderstadt (DE); ZARLING, Sven, 37115 Duderstadt-Immingerode (DE)
(74) Vertreter: Lins, Edgar
(86) Internationale Anmeldenummer: PCT/DE2007/000057
(87) Internationale Veröffentlichungsnummer: WO 2007/085228

(56) Entgegenhaltungen:
- WO-A-02/02034
- WO-A-91/15171
- WO-A-2005/117749
- FR-A- 2 048 707
- US-A- 4 446 580
- US-A- 5 993 488
- US-A1- 2002 183 860
- US-A1- 2004 162 623

## Beschreibung

Die Erfindung betrifft einen künstlichen Fuß mit einer sich von einem Fersenbereich zu einem Zehenbereich erstreckenden Längsachse, einer Länge, einer Breite und einer Höhe, einem Anschluss zu einem Unterschenkelteil, einer in Richtung der Höhe oberen Stützstruktur, einer sich vom Fersenbereich in den Zehenbereich erstreckenden elastischen Sohlenstruktur und einem zwischen der oberen Stützstruktur und der Sohlenstruktur angeordneten elastischen Verbindungselement, wobei die obere Stützstruktur etwa in der Mitte des Fußes bezüglich seiner Länge mit der Sohlenstruktur mittels einer Kopplungsanordnung verbunden ist, die eine relative Kippbewegung zwischen Stützstruktur und Sohlenstruktur erlaubt.

Die Erfindung betrifft ferner ein Verfahren zur Steuerung der Bewegung eines künstlichen Fußes in Abhängigkeit von der Bewegung eines Unterschenkelteils eines Patienten.

Es ist bekannt, einen künstlichen Fuß an einem Unterschenkelteil eines Patienten so anzubringen, dass der Fußaufbau ohne ein Gelenk im Bereich des natürlichen Knöchelgelenks auskommt. Die Abrollbewegung des Fußes beim Gehen wird dabei durch entsprechende elastische Ausbildungen des Fußaufbaus erreicht.

Durch US 5,993,488 ist ein Fußaufbau bekannt, der aus einer oberen Anschlussplatte, einer fest mit der Anschlussplatte verbundenen Knöchelplatte und einer etwas gewölbt geformten Sohlenplatte besteht. Die Sohlenplatte weist dabei etwa die Länge des künstlichen Fußes auf und ist in Längsrichtung flexibel, Die Knöchelplatte ist kürzer ausgebildet und liegt im Wesentlichen parallel zur Sohlenplatte. Zwischen der Knöchelplatte und der Sohlenplatte befindet sich ein Schaumstoffblock aus einem relativ festen, jedoch elastischen Schaumstoff. Der Schaumstoffblock weist eine Querschnittsfläche für einen horizontalen Schnitt auf, der der Querschnittsfläche der Knöchelplatte etwa entspricht, sodass der Schaumstoffblock den Zwischenraum zwischen Knöchelplatte und Sohlenplatte über die gesamte Größe der Knöcheiplatte ausfüllt. Die durch Klebung miteinander verbundenen Teile werden durch ein um die Sohlenplatte und die Knöchelplatte mit dem dazwischen liegenden Schaumstoffblock geschlungenes Band gegen eine Delaminierung gesichert. Dieser Aufbau des Fußes ermöglicht eine Komprimierung des Schaumstoffblocks, sodass durch eine Gewichtsverlagerung von hinten nach vorn eine Verschiebung der Kraftübertragung von der Knöchelplatte auf die Sohlenplatte in entsprechender Weise erfolgt. Dies kann für den Gehvorgang, also für das Abrollen über den Fuß, sinnvoll sein, führt jedoch zu einem unsicheren Gefühl beim Stehen, wenn lelchte Gewichtsverlagerungen zu entsprechenden Verlagerungen der Kraftelnleitung zwischen Knöchelplatte und Sohlenplatte führen. Es entsteht für den Patienten dabei ein unsicheres "schwimmendes" Gefühl, da kein definierter Punkt für die Einleitung der Gewichtskräfte in die Fußkonstruktion existiert. Dies gilt auch für seitliche Gewichtsverlagerungen und für Gewichtsverlagerungen in Zwischenrichtungen schräg zur Längsachse des Fußes.

Durch die US 4,446,580 ist eine Fußkonstruktion bekannt, bei der ein rohrförmiges Unterschenkelteil über ein bezüglich seiner Längsachse nach vorn versetztes Drehgelenk mit einer Basisplatte verbunden ist. Das Unterschenkelteil wird an relativ zu einer gelenkig an der Basisplatte befestigten Führungsstange geführt, wobei die Schwenkbewegung des Unterschenkelteils um das Drehgelenk in der Basisplatte mittels plastischer Dämpfer bedämpft wird. Die Basisplatte ist fest in einer Ausnehmung eines massiv ausgebildeten künstlichen Fußes verankert. Eine elastisch federnde Sohlenstruktur und eine damit verbundene Fersenauftrittsdämpfung sind nicht vorgesehen.

Durch WO 2005/117749 A2 ist eine ähnliche Konstruktion bekannt, bei der ein oberes Stützelement in Form einer Platte mit einem längeren Sohlenelement über ein keilförmiges Knöchelteil verbunden ist. Das keilförmige Knöchelteil besteht aus einem elastischen Material und bedämpft die Relativbewegung zwischen dem oberen Strukturelement und dem Sohlenelement. Etwa in der Fußmitte befindet sich ein Schlitz, mit der einer Übertragung einer Kompression im Fersenbereich auf eine entsprechende Zugkraft im Zehenbereich unterbunden werden kann. Im Zehenbereich entsteht ein Kontaktbereich, dessen vordere Kante als Drehmoment für das Fußelement fungiert. Die wirksame vordere Kante wandert dabei bei einer sich nach vorn ändernden Belastung nach vorn ab.

WO 02/02034 A1 offenbart einen Fußaufbau mit einem langgestreckten, sich vom Fersenbereich zum Zehenbereich erstreckenden Sohlenteil und einen oberen Fußteil in Form eines liegenden U oder L, wobei ein federnder Schenkel des oberen Fußteils im Wesentlichen horizontal verläuft. Zwischen dem Sohlenteil und dem oberen Fußteil befindet sich ein energieübertragendes Medium, das eine elastisch bedämpfte Bewegung der beiden Teile des Fußaufbaus zueinander ermöglicht. Das Medium kann dabei als Doppelkeil ausgebildet sein, wobei sich die beiden verbreiterten Enden der Keile nach vorn bzw. nach hinten erstrecken, sodass die dünneren Keilenden zueinander zeigen. Die beiden Keile können in einem mittleren Bereich miteinander verbunden oder in einem mittleren Bereich durch einen Abstand voneinander getrennt sein. Die Doppelkeile erstrecken sich über einen wesentlichen Abschnitt der Fußlänge, sodass eine Gewichtsverlagerung im Stehen auch zu einer Verlagerung des Krafteinleitungspunktes für die Übertragung der Bewegung des oberen Fußteils auf das Sohlenteil führt. Während diese Art der Verbindung der beiden Fußteile miteinander eine mehrachsige Bewegung ermöglicht, ist in einem weiteren Ausführungsbeispiel eine Gelenkverbindung zwischen dem oberen Fußteil- und dem Sohlenteil vorgesehen. Dadurch wird eine einzige Drehachse festgelegt, sodass eine mehrachsige gelenkige Verbindung nicht mehr besteht.

Der vorliegenden Erfindung liegt die Aufgabe zugrunde, eine Bewegung des künstlichen Fußes zu ermöglichen, die einerseits ein gleichmäßiges Abrollen ermöglicht und andererseits auch für den Stand ein sicheres Gefühl vermittelt.

Zur Lösung dieser Aufgabe ist ein künstlicher Fuß der eingangs erwähnten Art, bei dem die Kopplungsanordnung den Abstand zwischen Stützstruktur und Sohlenstruktur in der Mitte der Kopplungsanordnung zumindest bei einer Gewichtsbelastung im Stand des Patienten konstant hält und die Übertragung der Kräfte des Unterschenkelteils auf die Sohlenstruktur dadurch an einer definierten, unverändert bleibenden Einleitungsstelle in der Fußmitte erfolgt, dadurch gekennzeichnet, dass die obere Stützstruktur aus einem Material besteht, dessen Elastizität deutlich geringer ist als die Elastizität der Sohlenstruktur.

Zur Lösung der Aufgabe ist ferner ein Verfahren der eingangs erwähnten Art dadurch gekennzeichnet, dass die Bewegung des Unterschenkelteils mittels einer Stützstruktur in eine elastische Sohlenstruktur an einer definierten Einleitungsstelle in einem auf die Länge bezogenen Mittenbereichs des Fußes mehrachsig gelenkig so eingeleitet wird, dass zumindest beim Stehen des Patienten keine Änderung des Abstandes zwischen Stützstruktur und Sohlenstruktur an der unverändert bleibenden Einleitungsstelle eintritt und dass im Fersenbereich die Bewegung der elastischen Sohlenstruktur in Richtung auf die Stützstruktur elastisch bedämpft und in Richtung von der Stützstruktur weg begrenzt wird.

Erfindungsgemäß wird die Übertragung der Kräfte des Unterschenkelteils auf die Sohlenstruktur an einer definierten Einleitungsstelle vorgenommen, an der die Stützstruktur mit der Sohlenstruktur mehrachsig gelenkig so verbunden ist, dass eine relative Kippbewegung zwischen Stützstruktur und Sohlenstruktur elastisch bedämpft möglich ist, der Abstand zwischen Stützstruktur und Sohlenstruktur in der Mitte der Kopplungsanordnung an der Einleitungsstelle zumindest beim Stehen des Patienten unverändert bleibt, sodass die Krafteinleitung unverändert an der Einleitungsstelle, d.h. in der Fußmitte, verbleibt.

Dies gilt prinzipiell auch für den Abrollvorgang beim Gehen. Dabei ist es allerdings möglich, durch eine Abstandsverringerung durch höhere, beim Gehen auftretende Kompressionskräfte an der Einleitungsstelle eine geringfügige Verlagerung des Krafteinleitungspunktes nach hinten zu ermöglichen, da der zwischen der Stützstruktur und der Sohlenstruktur wirksame Kraftvektor durch die Kippbewegung der Stützstruktur nach vorne etwas nach hinten wandert. Dies führt zu einem komfortablen Abrollvorgang und zu einem Gefühl erhöhter Sicherheit beim Abrollen.

In einer bevorzugten Ausführungsform des erfindungsgemäßen künstlichen Fußes ist die Kopplungsanordnung als Gelenkanordnung ausgebildet und beispielsweise durch ein Materialstück gebildet, das durch Kippmomente auf einem Teil seines Querschnitts komprimierbar ist, jedoch durch die beim Stehen auftretende Kraft nicht in seiner Länge komprimiert wird. Diese Komprimierung, die zu einer Verringerung der Länge des Zylinders führt, wird nur durch höhere Kräfte, wie sie beim Gehvorgang auftreten, zugelassen. Das Materialstück hat vorzugsweise die Form eines Zylinders mit einem beliebigen Querschnitt, vorzugsweise die Form eines Kreiszylinders.

Das elastische Verbindungselement ist bei dem erfindungsgemäßen künstlichen Fuß nur im Fersenbereich angeordnet, erstreckt sich also regelmäßig nicht über die gesamte Länge der Stützstruktur. Das elastische Verbindungselement kann eine gebogene Blattfeder oder eine andere Federanordnung sein, ist jedoch bevorzugt ein elastisches Polster aus einem Kunststoff-Schaummaterial.

Die Begrenzungseinrichtung ist vorzugsweise ein flexibles und vorzugsweise unelastisches Band, das bei zunehmendem Abstand der oberen Stützstruktur von der Sohlenstruktur spannbar ist, also insbesondere beim Abrollen des künstlichen Fußes über den Zehenbereich.

Das flexible Band ist dabei vorzugsweise mit einer Umlenkung durch das Polster geführt, sodass zum Spannen des Bandes eine elastische Verdrängung von Material des Polsters erforderlich ist. Somit ist es möglich, den Vorfußwiderstand beim Abrollen über den Vorfuß durch die Führung des flexiblen Bandes durch das Polster bzw. um das Polster herum zu steuern und so auf die Bedürfnisse des Patienten anzupassen.

Die obere Stützstruktur besteht vorzugsweise aus einem starren Material, erfindungsgemäß zumindest aber aus einem Material, dessen Elastizität deutlich geringer ist als die Elastizität der Sohlenstruktur.

In einer bevorzugten Ausführungsform des erfindungsgemäßen Fußes ist die Gelenkanordnung am zehenseitigen Ende der oberen Stützstruktur angeordnet. Anders ausgedrückt endet die obige Stützstruktur zweckmäßigerweise an der Gelenkanordnung.

In einer konstruktiv bevorzugten Ausführungsform weist die obere Stützstruktur eine Unterseite auf, die von dem zehenseitigen Ende relativ zu der Sohlenstruktur ansteigt, sodass sie am fersenseitigen Ende einen größeren Abstand zur Sohlenstruktur aufweist als im Bereich der Kopplungsanordnung.

Für eine einfache Montage des erfindungsgemäßen künstlichen Fußes ist es zweckmäßig, wenn das elastische Polster einen horizontalen Schlitz aufweist, in den die Sohlenstruktur eingeschoben ist. Das die Begrenzungseinrichtung bildende flexible Band ist vorzugsweise um die Unterseite der Sohlenstruktur geschlungen. Die obere Stützstruktur kann dann vorzugsweise ein von dem Band an seiner Oberseite umschlingbaren Ansatz aufweisen.

Der erfindungsgemäße künstliche Fuß erlaubt ferner eine stabile Stehfunktion auch mit unterschiedlichen Absatzhöhen für einen mit dem künstlichen Fuß getragenen Schuh, wenn in einer bevorzugten Ausführungsform kraftflussmäßig in Reihe mit dem elastischen Verbindungselement eine verriegelbare Höhenverstelleinrichtung vorgesehen ist, mit der der Abstand zwischen der Sohlenstruktur und der oberen Stützstruktur einstellbar ist. Mittels der Höhenverstelleinrichtung kann der Winkel zwischen der oberen Stützstruktur und der Sohlenstruktur eingestellt werden, wodurch eine Anpassung an unterschiedliche Absatzhöhen möglich ist.

Die Erfindung soll im Folgenden anhand von in der Zeichnung dargestellten Ausführungsbeispielen näher erläutert werden. Es zeigen:
- Figur 1: eine schematische Darstellung des konstruktiven Aufbaus eines erfindungsgemäßen künstlichen Fußes in einer ersten Ausführungsform;
- Figur 2: ein Ausführungsbeispiel für die Anordnung eines als Begrenzungseinrichtung dienenden flexiblen, jedoch unelastischen Bandes;
- Figur 3: eine modifizierte Anordnung des Bandes zur Steuerung des Vorfußwiderstandes beim Abrollen;
- Figur 4: eine schematische Darstellung gemäß Figur 1 für eine zweite Ausführungsform eines erfindungsgemäßen künstlichen Fußes;
- Figuren 5 bis 7: drei Phasen für die Montage des künstlichen Fußes gemäß Figur 1,
- Figur 8: eine schematische Darstellung einer dritten Ausführungsform eines erfindungsgemäßen künstlichen Fußes;
- Figur 9: eine schematische Darstellung einer vierten Ausführungsform eines erfindungsgemäßen künstlichen Fußes;
- Figur 10: eine schematische Darstellung einer fünften Ausführungsform eines erfindungsgemäßen künstlichen Fußes und
- Figur 11: eine schematische Darstellung einer sechsten Ausführungsform eines erfindungsgemäßen künstlichen Fußes;
- Figur 12: zwei schematische Darstellungen einer siebten Ausführungsform eines erfindungsgemäßen künstlichen Fußes, die mit einer Höhenverstelleinrichtung versehen ist.

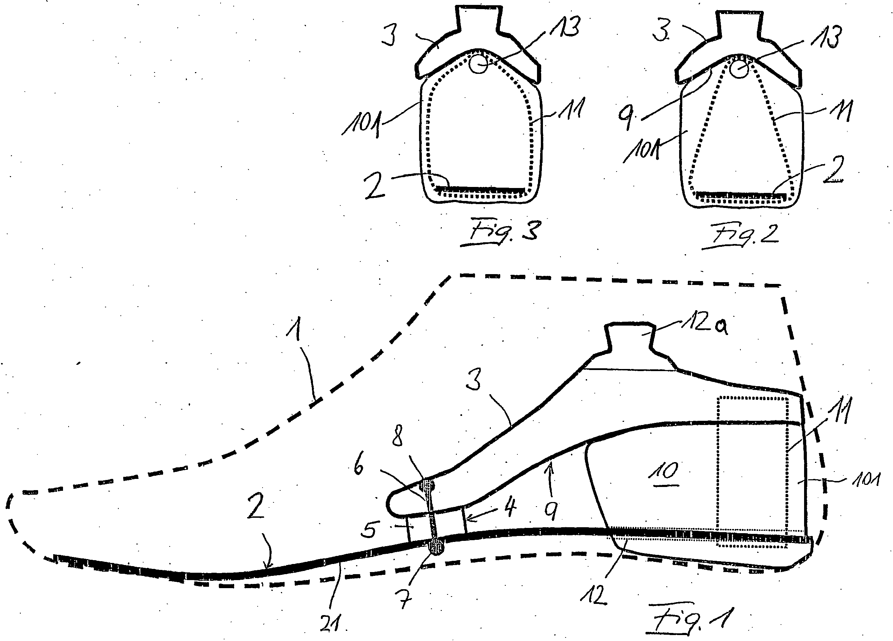

Figur 1 lässt in gestrichelter Darstellung die Umrisse einer kosmetischen Hülle 1 eines künstlichen Fußes erkennen. Im Wesentlichen über die gesamte Länge des Fußes erstreckt sich eine Sohlenstruktur 2, die in einem Zehenbereich von unten gesehen eine konvexe Wölbung aufweist, die etwa in der Mitte des Fußes in eine konkave Wölbung übergeht und sich bis zu einem Fersenbereich erstreckt. Die Sohlenstruktur 2 besteht in dem dargestellten Ausführungsbeispiel aus einer flächigen, streifenförmigen, langgestreckten Sohlenfeder 21. Etwa in der Mitte des Fußes ist die Sohlenstruktur 2 mit einem vorderen Ende einer Stützstruktur 3 über eine durch eine Gelenkanordnung 4 gebildete Kopplungsanordnung verbunden. Die Gelenkanordnung 4 besteht aus einem Materialstück 5 in Form eines Kunststoffzylinders, der sich mit einer unteren Stirnfläche an der Sohlenstruktur 2 und mit einer oberen Stirnfläche an der Unterseite der Stützstruktur 3 abstützt. In der dargestellten Ausführungsform ist die Gelenkanordnung 4 durch eine Stiftanordnung 6 ergänzt, die an ihren beiden Enden Kugelköpfe 7, 8 aufweist. Die Kugelköpfe 7, 8 stützen sich an der Sohlenstruktur 2 einerseits und an der Stützstruktur 3 andererseits ab und erhöhen die Stabilität der Verbindung zwischen der Stützstruktur 3, dem Zylinder 5 und der Sohlenstruktur 2, ohne eine relative Kippbarkeit von Stützstruktur 3 relativ zu der Sohlenstruktur 2 zu behindern.

Die relative Kippbarkeit ergibt sich aus einer elastischen Ausbildung des Zylinders 5 derart, dass der Zylinder 5 durch Schrägstellungen der Stützstruktur 3 relativ zur Sohlenstruktur 2 auf einem Teil seines Querschnitts durch die Gewichtskräfte des Patienten eindrückbar ist, wobei der im Querschnitt diametral gegenüber liegende Teil des Zylinders 5 sich ausdehnt, sodass die mittlere Höhe des Zylinders 5, also der resultierende mittlere Abstand zwischen Stützstruktur 3 und Sohlenstruktur 2 im Bereich der Gelenkanordnung 4 konstant bleibt.

Eine Verringerung des mittleren Abstandes wird aufgrund der Ausbildung des Zylinders 5 nur durch höhere Kräfte zugelassen, wie sie an der Gelenkanordnung 4 beim Abrollen des Fußes während des Gehvorganges auftreten.

Die Stützstruktur 3 weist eine Unterseite 9 auf, die von der Gelenkanordnung 4 zum Fersenbereich relativ zur Sohlenstruktur 2 ansteigend ausgebildet ist, sodass der Abstand zwischen Stützstruktur 3 und Sohlenstruktur 2 ausgehend von der Gelenkanordnung 4 zum Fersenbereich hin zunimmt. Im Fersenbereich ist zwischen der Stützstruktur 3 und der Sohlenstruktur 2 ein elastisches Verbindungselement 10 in Form eines elastischen Polsters 101 angeordnet. In dem elastischen Polster 101 ist eine Begrenzungseinrichtung in Form eines flexiblen Bandes 11 geführt, durch das ein maximaler Abstand zwischen Stützstruktur 3 und Sohlenstruktur 2 im Bereich des Bandes 11 festgelegt wird, wie anhand der Figuren 2 und 3 noch näher erläutert wird.

Die Stützstruktur weist an ihrer Oberseite einen Justierzapfen 12a auf, mit dem eine Verbindung zu einem entsprechenden Unterschenkelteil einer Unterschenkelprothese befestigbar ist.

Figur 2 lässt erkennen, dass das flexible, aber unelastische Band innerhalb des elastischen Polsters 101 geführt ist und die Unterseite der Sohlenstruktur 2 umschlingt Zur Aufnahme der Sohlenstruktur 2 ist das elastische Polster 101 mit einem durchgehenden horizontalen Schlitz 12 versehen, der in Figur 1 gepunktet dargestellt ist.

Das endlos ausgebildete Band 11 umschlingt einen zylindrischen Ansatz 13 an der Unterseite der Stützstruktur 2.

Figur 2 zeigt den gespannten Zustand des Bandes 11 im Zustand des maximalen Abstandes zwischen Stützstruktur 3 und Sohlenstruktur 2. Es ist erkennbar, dass bei einem Fersenauftritt das elastische Polster 101 komprimierbar ist, wodurch das flexible Band 11 aus dem gespannten Zustand in einen Falten werfenden, schlaffen Zustand übergeht. Bei der anschließenden Expansion des elastischen Polsters 101 bzw. bei der Belastung des Vorfußbereiches der Sohlenstruktur 2 wird das flexible Band 11. wieder in die in Figur 2 dargestellte gespannte Position verbracht.

Der Figur 2 ist ferner zu entnehmen, dass die Unterseite 9 der Stützstruktur 3 dachförmig ausgebildet ist und dass das elastische Polster 101 mit einer entsprechenden dachförmigen Oberseite an der dachförmigen Unterseite 9 der Stützstruktur 3 anliegt und dort beilspielsweise verklebt werden kann.

Bei dem in Figur 3 dargestellten Ausführungsbeispiel verläuft das Band 11 zwischen der Sohlenstruktur 2 und dem Ansatz 13 innerhalb des elastischen Polsters auf einer gebogenen Linie. Zur Ausübung der Begrenzungsfunktion für den maximalen Abstand zwischen Stützstruktur 3 und Sohlenstruktur 2 wird das Band 11 so gespannt, dass es zwischen dem Ansatz 13 und den seitlichen Kanten der Sohlenstruktur 2 eine gerade Linie ausbildet. Hierfür muss das Band 11 Bereiche des elastischen Polsters 101 seitlich komprimieren, sodass eine elastische Bedämpfung der Abstandsvergrößerung zwischen Sohlenstruktur 2 und Stützstruktur 3 bei einer Vorfußbelastung der Sohlenstruktur 2 entsteht. Die Abrollbewegung über den Vorfuß der Sohlenstruktur 2 wird somit mit einem erhöhten Widerstand möglich, sodass durch die Anordnung des flexiblen Bandes 11 eine Steuerung des Vorfußwiderstandes des künstlichen Fußes möglich ist.

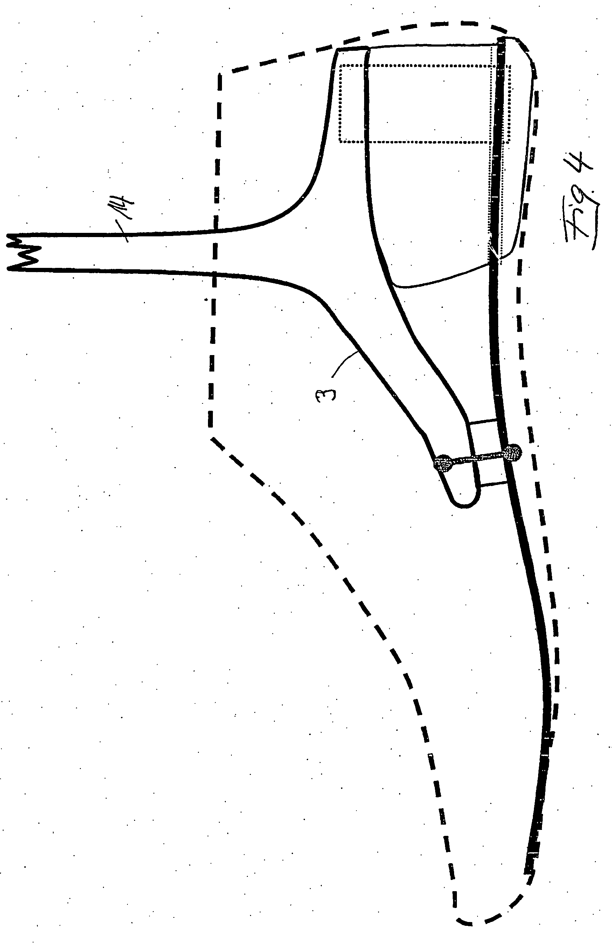

Die in Figur 4 dargestellte Ausführungsform unterscheidet sich von der in Figur 1 dargestellten Ausführungsform nur dadurch, dass die Stützstruktur 3 einstückig in ein Unterschenkelteil 14 übergeht, sodass keine Verbindung zwischen Unterschenkelteil 14 und Stützstruktur 3 hergestellt werden muss.

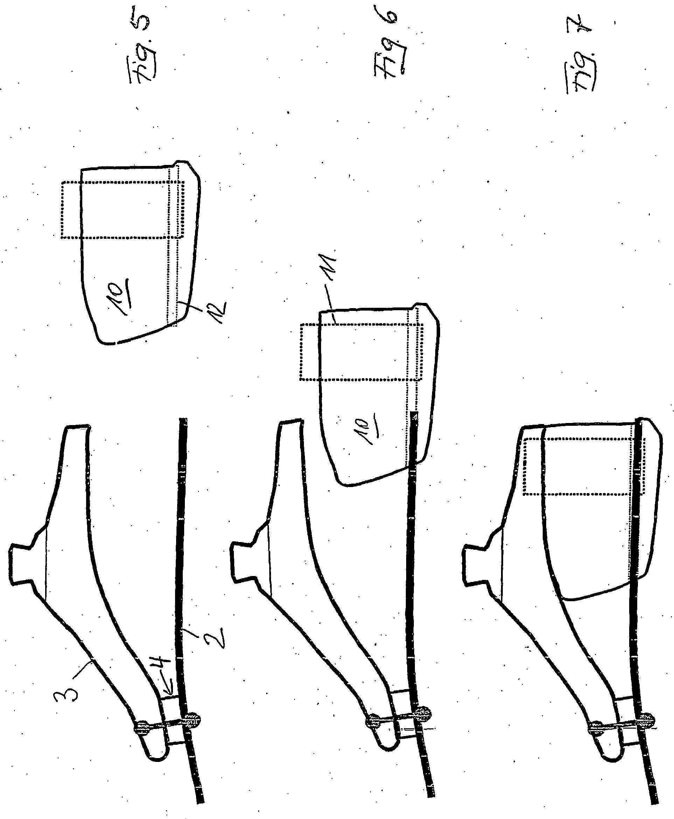

Die Montage des erfindungsgemäßen künstlichen Fußes erfolgt durch Verbindung der Stützstruktur 3 mit der Sohlenstruktur 2 über die Gelenkanordnung 4, wodurch sich ein nach hinten öffnender Fersenbereich ergibt. In den konisch geöffneten Zwischenraum zwischen Stützstruktur 3 und Sohlenstruktur 2 wird das elastische Polster 101 eingeführt, indem die Sohlenstrüktur 2 mit ihrem hinteren Ende in den zur Aufnahme dienenden Schlitz 12 eingeschoben wird, wie dies Figur 6 in einer Zwischenstellung zeigt. Beim weiteren Einschieben des elastischen Polsters 101 wird das flexible Band 11, das aus der Oberseite des elastischen Polsters 101 mit einer Schlinge herausragt, über den in den Figuren 5 bis 7 nicht dargestellten Ansatz 13 geschoben. Nach dem vollständigen Aufschieben des elastischen Polsters 101 unter Montage der Begrenzungseinrichtung in Form des geschlossen umlaufenden flexiblen, aber unelastischen Bandes 11 kann das elastische Polster 101 durch eine aushärtende Verklebung mit der Stützstruktur 3 und ggf. der Sohlenstruktur 2 verbunden werden.

Die dachförmige Ausbildung der Unterseite 9 der Stützstruktur 3 und die entsprechende dachförmige Ausbildung der Oberseite des elastischen Polsters 101 bewirkt eine automatische Zentrierung des elastischen Polsters 101 in Verbindung mit der durch das Einschieben der Sohlenstruktur 2 in den Aufnahmeschlitz 12 bewirkten Führung.

Figur 7 lässt - ebenso wie Figur 1 und Figur 4 - erkennen, dass der unter die Sohlenstruktur 2 ragende Teil des elastischen Polsters 101 als Auftrittsdämpfer für die Sohlenstruktur 2 beim Fersenauftritt dienen kann. Eine etwaige Formgebung des elastischen Polsters 101 an dieser Unterseite begünstigt den Abrollvorgang beim Fersenauftritt.

Die in den dargestellten Ausführungsbeispielen durch den Zylinder 5 gebildete Gelenkanordnung kann auch in anderer Weise ausgeführt werden, beispielsweise in Form einer Kugelkalotte, die mit einer entsprechenden Kugelpfanne zusammenwirkt, um so die kippbare Verbindung zwischen Stützstruktur 3 und Sohlenstruktur 2 herzustellen, ohne dabei den Abstand zwischen Stützstruktur 3 und Sohlenstruktur 2 an der Einleitungsstelle der Kraft, also im Bereich der Gelenkanordnung 4, beim Stehen des Patienten zu verändern. Aufgrund der höheren Kräfte beim Gehvorgang kann beispielsweise die Kugelkalotte aus einem durch diese Kräfte etwas eindrückbaren Kunststoff oder Hartgummi gebildet sein.

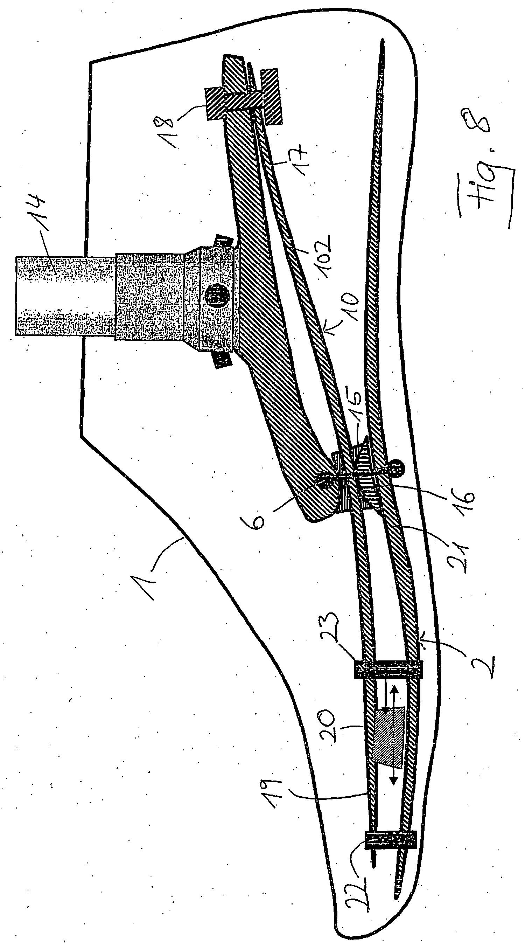

Die in Figur 8 dargestellte dritte Ausführungsform eines erfindungsgemäßen künstlichen Fußes weist ein elastisches Verbindungselement 10 zwischen Stützstruktur 3 und Sohlenstruktur 2 in Form einer Steuerfeder 102 auf. Die Steuerfeder ist als zweiarmiges Federteil ausgebildet und bildet mit einem Mittelstück 15 einen Teil der Gelenkanordnung 4. Dazu ist das Mittelstück an seiner Oberseite mit einer konkaven Wölbung versehen, in die eine entsprechende konvexe Wölbung des zehnseitigen Endes der Stützstruktur 3 aufliegt. Die Unterseite des Mittelstücks 15 ist zur Unterseite ebenfalls konkav gewölbt und liegt an einer entsprechenden konvexen Wölbung eines Kunststoffdämpfers 16 an. Die Gelenkanordnung 4 wird durch eine Stiftanordnung 6 zusammengehalten.

Die Steuerfeder 102 ist mit einem fersengerichteten Arm 17 am fersenseitigen Ende der Stützstruktur 3 mittels einer Feststellschraube 18 verbunden. Ein zehengerichteter Arm 19 der Steuerfeder 102 verläuft etwa parallel zum zehenseitigen Ende der Sohlenfeder 21. In diesem Bereich sind der zehengerichtete Arm 19 der Steuerfeder 102 und die Sohlenfeder 21 über einen elastischen Dämpfer 20 und zwei beiderseits des Dämpfers 20 angeordnete flexible, aber unelastische Haltebänder 22, 23 verbunden.

Beim Fersenauftritt während des Gehvorganges wird der fersengerichtete Arm 17 der Steuerfeder 102 nach unten, d.h. in Richtung auf das fersenseitige Ende der Sohlenfeder 21 elastisch verformt. Neben der den Fersenauftritt dämpfenden Verformung des fersengerichteten Arms 17 entsteht ein nach oben gerichtetes Drehmoment auf den zehengerichteten Arm 19 der Steuerfeder 102. Während der Fuß eine Plantarflexion bezüglich des Unterschenkelteils 14 ausführt, wird der Zehenbereich des Fußes durch den zehengerichteten Arm 19 der Steuerfeder 102 angehoben, wodurch das Abrollen des Fußes erleichtert wird.

Das flexible Halteband 23, das zwischen Mittelstück 15 und dem elastischen Dämpfer 20 angeordnet ist, ist ebenso wie der elastische Dämpfer 20 in Längsrichtung verstellbar, wie dies die Pfeile in Figur 8 verdeutlichen. Eine Verschiebung des flexiblen Haltebands 23 von der hier dargestellten Mitte zwischen Mittelstück 15 und zehenseitigem Ende der Sohlenstruktur 2 führt zu einer Reduzierung der resultierenden Härte des fersengerichteten Arms 17 der Steuerfeder 102. Der Fersenauftritt wird weicher bedämpft. Unabhängig davon wirkt die Position des elastischen Dämpfers 20 als Einstellmittel der Vorfußhärte der Sohlenstruktur 2. Mit Annäherung des elastischen Dämpfers and die Vorfußspitze wird der Vorfuß steifer.

Die Sohlenfeder 21 und die Steuerfeder 102 sind so abgestimmt, dass bei einem Fersenauftritt die gewünschte Plantarflexion erfolgt.

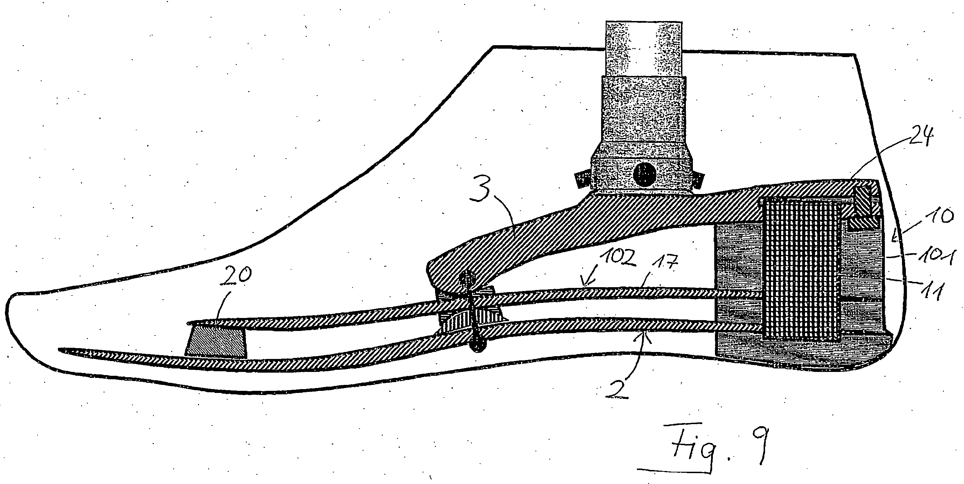

Bei dem in Figur 9 dargestellten vierten Ausführungsbeispiel ist das elastische Verbindungselement 10 wiederum im Wesentlichen durch das elastische Polster 101 im Fersenbereich gebildet. Das flexible Band 11, das den maximalen Abstand zwischen dem hinteren Ende der Stützstruktur 3 und dem fersenseitigen Ende der Sohlenstruktur 2 begrenzt, ist durch einen nach hinten offenen Schlitz 24 der Stützstruktur 3 verschlungen. Die hier vorhandene Steuerfeder 102 ragt mit ihrem fersengerichteten Arm 17 in das elastische Polster 101 hinein. In diesem Fall bildet die Steuerfeder 102 im Wesentlichen einen Teil der Sohlenstruktur 2. Durch die Dimensionierung und Positionierung des elastischen Dämpfers 20 lässt sich die Vorfußsteifigkeit einstellen.

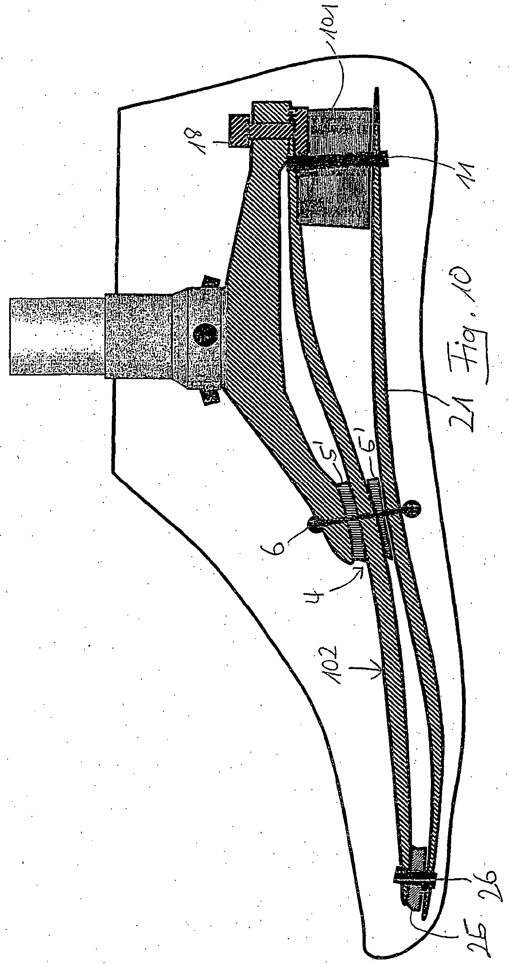

Bei der in Figur 10 dargestellten fünften Ausführungsform ist die Steuerfeder 102 mit ihrem fersenseitigen Ende wiederum über die Feststellschraube 18 mit dem fersenseitigen Ende der Stützstruktur 3 verbunden. Zusätzlich ist zwischen der Stützstruktur 3 bzw. dem fersengerichteten Arm 17 der Steuerfeder 102 das elastische Polster 101 zur Fersenauftrittsdämpfung angeordnet. Die den Abstand vergrößernde Bewegung zwischen Stützstruktur 3 und Sohlenstruktur 2 im Fersenbereich wird durch das flexible Band 11 begrenzt.

Das Drehgelenk 4 ist im vorliegenden Fall, ähnlich wie bei dem in Figur 1 dargestellten Ausführungsbeispiel, durch eine Stiftanordnung 6 und, hier zwei, Kunststoffzylinder 5' gebildet, die zwischen der Stützstruktur 3 und der Steuerfeder 102 einerseits und zwischen der Steuerfeder 102 und der Sohlenfeder 21 andererseits angeordnet sind.

Bei dieser Ausführungsform sind die vorderen Enden der Steuerfeder 102 und der Sohlenfeder 21 durch einen elastischen Dämpfer 25 und ein flexibles, aber unelastisches Halteband 26 miteinander verbunden. Auch hier sind Steuerfeder 102 und Sohlenfeder 21 so aufeinander abgestimmt, dass bei Fersenauftritt eine Plantarflexion erfolgt. Die Fersensteifigkeit kann durch Auswechseln des elastischen Polsters 101 eingestellt werden.

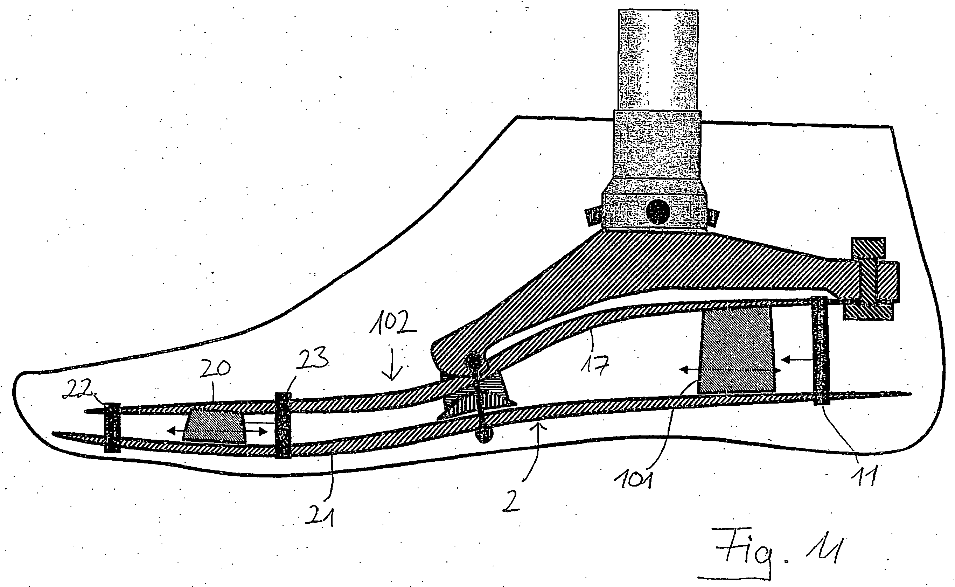

Figur 11 zeigt ein gegenüber Figur 10 abgewandeltes Ausführungsbeispiel, bei dem verbesserte Einstellmöglichkeiten vorgesehen sind. Wie bei dem in Figur 8 dargestellten Ausführungsbeispiel kann durch ein Verschieben des flexiblen Haltebandes 23 zum fersenseitigen Ende der Sohlenstruktur 2 hin die durch den fersengerichteten Arm 17 der Steuerfeder 102 bewirkte Dämpfung weicher eingestellt werden. Die Verschiebung des elastischen Dämpfers 20 in Längsrichtung bewirkt eine Veränderung der Vorfußhärte, wobei durch eine Verschiebung des elastischen Dämpfers zum Zehenende hin eine größere Härte des Vorfußes eingestellt wird. Durch eine Verschiebung des plastischen Polsters 101 in Längsrichtung und des zugehörigen flexiblen Bands 11 lässt sich zusätzlich die Dämpfung des Fersenauftritts einerseits und die Vorfußhärte andererseits beeinflussen, weil der wirksame fersengerichtete Arm 17 der zweiarmigen Steuerfeder 102 in seiner Länge verändert wird.

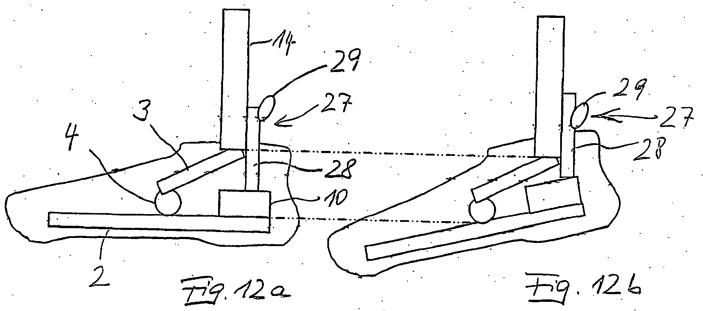

In den Figuren 12a und 12b ist eine siebte Ausführungsform eines erfindungsgemäßen künstlichen Fußes schematisch dargestellt. Die Darstellung lässt schematisch die Sohlenstruktur 2, das Drehgelenk 4, die obere Stützstruktur 3 und ein Unterschenkelteil 14 erkennen. Das Unterschenkelteil 14 ist mit der oberen Stützstruktur 3 starr verbunden. Im Fersenbereich ist das elastische Verbindungselement 10, insbesondere in Form eines Fersenpuffers, vorgesehen. Dieses ist mit der oberen Stützstruktur 3 bzw. dem Unterschenkelteil 14 nicht unmittelbar, sondern vielmehr über einen Stempel 28 verbunden, der relativ zum Unterschenkelteil 14 verschiebbar angeordnet ist und mittels eines schematisch dargestellten Verriegelungselements 29 feststellbar ist.

Figur 12a zeigt das die siebte Ausführungsform in der so genannten Barfußstellung, also ohne eine zusätzliche Absatzhöhe eines Schuhs. Figur 12b zeigt demgegenüber, dass die Sohlenstruktur 2 in ihrem Abstand zum Unterschenkelteil 14 bzw. der oberen Stützstruktur 3 verringert worden ist, sodass die Sohlenstruktur 2 in ihrer Winkelstellung relativ zum Unterschenkelteil 14 und der oberen Stützstruktur 3 so verändert worden ist, dass eine Anpassung an eine Absatzhöhe eines Schuhs gewährleistet ist. Es ist ohne weiteres ersichtlich, dass durch unterschiedliche Verstellungen des Stempels 28 relativ zum Unterschenkelteil 14 bzw. zur oberen Stützstruktur 3 Anpassungen an unterschiedliche Absatzhöhen möglich sind.

Die erfindungsgemäße Konstruktion ermöglicht eine komfortable Abrollbewegung mit den natürlichen Fuß entsprechenden bedämpften Kippbewegungen, ohne aufgrund von Änderungen des Krafteinleitungspunktes beim Stehen Unsicherheiten für den Patienten hervorzurufen. Die erfindungsgemäßen Konstruktionen lassen aufgrund des unveränderten mittleren Abstandes zwischen der Stützstruktur 3 und der Sohlenstruktur 2 im Bereich der Gelenkanordnung 4 den Krafteinleitungspunkt unverändert, jedenfalls wenn der Patient steht. Beim Gehen kann eine für den Abrollvorgang vorteilhafte geringe Rückverlagerung des Krafteinleitungspunktes realisiert werden.

## Patentansprüche

1. Künstlicher Fuß mit einer sich von einem Fersenbereich zu einem Zehenbereich erstreckenden Längsachse, einer Länge, einer Breite und einer Höhe, einem Anschluss zu einem Unterschenkelteil (14), einer in Richtung der Höhe oberen Stützstruktur (3), einer sich vom Fersenbereich in den Zehenbereich erstreckenden elastischen Sohlenstruktur (2) und einem zwischen der oberen Stützstruktur (3) und der Sohlenstruktur (2) angeordneten elastischen Verbindungselement (10), wobei die obere Stützstruktur (3) etwa in der Mitte des Fußes bezüglich seiner Länge mit der Sohlenstruktur (2) mittels einer Kopplungsanordnung (4) verbunden ist, die eine relative Kippbewegung zwischen Stützstruktur (3) und Sohlenstruktur (2) erlaubt, wobei die Kopplungsanordnung (4) derart ausgebildet ist, dass sie den Abstand zwischen Stützstruktur (3) und Sohlenstruktur (2) in der Mitte der Kopplungsanordnung (4) zumindest bei einer Gewichtsbelastung im Stand des Patienten konstant hält und wobei die Übertragung der Kräfte des Unterschenkelteils auf die Sohlenstruktur dadurch an einer definierten, unverändert bleibenden Einleitungsstelle etwa in der Fußmitte erfolgt **dadurch gekennzeichnet, dass** die obere Stützstruktur (3) aus einem Material besteht, dessen Elastizität deutlich geringer ist als die Elastizität der Sohlenstruktur.

2. Künstlicher Fuß nach Anspruch 1, **dadurch gekennzeichnet, dass** die Kopplungsanordnung (4) durch ein Materialstück (5) gebildet ist, das durch Kippmomente auf einem Teil seines Querschnitts komprimierbar ist.

3. Künstlicher Fuß nach Anspruch 1 oder 2, **dadurch gekennzeichnet, dass** die Kopplungsanordnung (4) durch höhere Kräfte, wie sie beim Gehvorgang auftreten, zur Verringerung des Abstands in der Mitte der Kopplungsanordnung (4) komprimierbar ist.

4. Künstlicher Fuß nach einem der Ansprüche 1 bis 3, **dadurch gekennzeichnet, dass** das elastische Verbindungselement (10) durch ein elastisches Polster (101) gebildet ist.

5. Künstlicher Fuß nach einem der Ansprüche 1 bis 4, **dadurch gekennzeichnet, dass** das elastische Verbindungselement (10) mit einer den Abstand zwischen Stützstruktur (3) und Sohlenstruktur (2) begrenzenden Begrenzungseinrichtung versehen ist.

6. Künstlicher Fuß nach Anspruch 5, **dadurch gekennzeichnet, dass** die Begrenzungseinrichtung durch ein flexibles Band (11) gebildet ist, das bei zunehmendem Abstand der oberen Stützstruktur (3) von der Sohlenstruktur (2) spannbar ist.

7. Künstlicher Fuß nach Anspruch 6, **dadurch gekennzeichnet, dass** das flexible Band (11) mit einer Umlenkung durch das Polster (101) geführt ist, sodass zum Spannen des Bandes (11) eine elastische Verdrängung von Material des Polsters (101) erforderlich ist.

8. Künstlicher Fuß nach einem der Ansprüche 1 bis 7, **dadurch gekennzeichnet, dass** die obere Stützstruktur (3) aus einem starren Material gebildet ist.

9. Künstlicher Fuß nach einem der Ansprüche 1 bis 8, **dadurch gekennzeichnet, dass** die Kopplungsanordnung (4) am zehenseitigen Ende der oberen Stützstruktur (3) angeordnet ist.

10. Künstlicher Fuß nach einem der Ansprüche 1 bis 9, **dadurch gekennzeichnet, dass** die obere Stützstruktur (3) eine Unterseite (9) aufweist, die von dem zehenseitigen Ende relativ zu der Sohlenstruktur (2) ansteigt, sodass sie am fersenseitigen Ende einen größeren Abstand zur Sohlenstruktur aufweist als im Bereich der Kopplungsanordnung (4).

11. Künstlicher Fuß nach einem der Ansprüche 4 bis 10, **dadurch gekennzeichnet, dass** das elastische Polster (101) einen horizontalen Schlitz (12) aufweist, in den die Sohlenstruktur (2) eingeschoben ist.

12. Künstlicher Fuß nach einem der Ansprüche 5 bis 11, **dadurch gekennzeichnet, dass** das Band (11) um die Unterseite der Sohlenstruktur (2) geschlungen ist.

13. Künstlicher Fuß nach Anspruch 12, **dadurch gekennzeichnet, dass** an der oberen Stützstruktur (3) ein von dem Band (11) an seiner Oberseite umschlingbarer Ansatz (13) angeordnet ist.

14. Künstlicher Fuß nach einem der Ansprüche 1 bis 13, **dadurch gekennzeichnet, dass** die Unterseite (9) der Stützstruktur (3) im Fersenbereich dachförmig ausgebildet ist.

15. Künstlicher Fuß nach Anspruch 14, **dadurch gekennzeichnet, dass** das elastische Polster (101) mit einer dachförmigen Oberseite an der dachförmigen Unterseite (9) der Stützstruktur (3) anliegt.

16. Künstlicher Fuß nach einem der Ansprüche 1 bis 15, **dadurch gekennzeichnet, dass** das elastische Verbindungselement (10) eine Steuerfeder (102) mit einem fersengerichteten Arm (17) aufweist.

17. Künstlicher Fuß nach einem der Ansprüche 1 bis 16, **dadurch gekennzeichnet, dass** ein zehengerichteter Arm (19) einer Steuerfeder (102) einen Teil der Sohlenstruktur (2) bildet, in dem der zehengerichtete Arm (19) mit dem vorderen Ende einer Sohlenfeder (21) der Sohlenstruktur (2) verbunden ist.

18. Künstlicher Fuß nach Anspruch 17, **dadurch gekennzeichnet, dass** die Verbindung des zehengerichteten Arms (19) mit der Sohlenfeder (21) über einen elastischen Dämpfer (20) erfolgt.

19. Künstlicher Fuß nach Anspruch 18, **dadurch gekennzeichnet, dass** in Längsrichtung beidseitig von dem elastischen Dämpfer (20) den Abstand zwischen Sohlenfeder (21) und dem zehengerichteten Arm (19) begrenzende flexible Haltebänder (22, 23) angeordnet sind.

20. Künstlicher Fuß nach einem der Ansprüche 4 bis 19, **dadurch gekennzeichnet, dass** das elastische Polster (101) in Längsrichtung verstellbar angeordnet ist.

21. Künstlicher Fuß nach einem der Ansprüche 1 bis 20, **dadurch gekennzeichnet, dass** kraftflussmäßig in Reihe mit dem elastischen Verbindungselement (10) eine verriegelbare Höhenverstelleinrichtung (27) vorgesehen ist, mit der der Abstand zwischen der Sohlenstruktur (2) und der oberen Stützstruktur (3) einstellbar ist.

22. Verfahren zur Steuerung der Bewegung eines künstlichen Fußes in Abhängigkeit von der Bewegung eines Unterschenkelteils (14) eines Patienten, **dadurch gekennzeichnet, dass** die Bewegung des Unterschenkelteils (14) mittels einer Stützstruktur (3) in eine elastische Sohlenstruktur (2) an einer definierten Einleitungsstelle in einem auf die Länge bezogenen Mittenbereichs des Fußes mehrachsig gelenkig so eingeleitet wird, dass zumindest beim Stehen des Patienten keine Änderung des Abstandes zwischen Stützstruktur (3) und Sohlenstruktur (2) an der unverändert bleibenden Einleitungsstelle eintritt und dass im Fersenbereich die Bewegung der elastischen Sohlenstruktur (2) in Richtung auf die Stützstruktur (3) elastisch bedämpft und in Richtung von der Stützstruktur (3) weg begrenzt wird.

23. Verfahren nach Anspruch 22, **dadurch gekennzeichnet, dass** eine Abrollbewegung im Zehenbereich durch die elastische Sohlenstruktur (2) kontrolliert wird.

24. Verfahren nach Anspruch 22 oder 23, **dadurch gekennzeichnet, dass** für ein Abrollen im Gehvorgang des Patienten eine Verringerung des Abstandes zwischen Stützstruktur (3) und Sohlenstruktur (2) an der Einleitungsstelle durch höhere Gewichtskräfte zugelassen wird.

## Claims

1. An artificial foot with a longitudinal axis extending from a heel area to a toe area, a length, a width and a height, a connection to a lower leg part (14), an upper supporting structure (3) in the direction of height, an elastic sole structure (2) extending from the heel area to the toe area and an elastic connecting element (10) arranged between the upper supporting structure (3) and the sole structure (2), wherein the upper supporting structure (3) is connected to the sole structure (2) approximately in the middle of the foot with respect to its length by means of a coupling system (4) which allows a relative tilting motion between supporting structure (3) and sole structure (2) wherein the coupling system (4) is arranged to keep the distance between supporting structure (3) and sole structure (2) in the middle of the coupling system (4) constant, at least while the patient places weight on it when standing, and wherein the transfer of forces of the lower leg part to the sole structure is made at a defined introductory point approximately in the centre of the foot, **characterized in that** the upper support structure (3) consists of a material having an elasticity clearly less than the elasticity of the sole structure.

2. The artificial foot as claimed in claim 1, **characterized in that** the coupling system (4) is formed by a material piece (5) which can be compressed by tilting moments on part of its cross-section.

3. The artificial foot as claimed in Claim 1 or 2, **characterized in that** the coupling system (4) can be compressed by higher forces, as occur during walking, for lessening the distance in the middle of the coupling system (4).

4. The artificial foot as claimed in any one of Claims 1 to 3, **characterized in that** the elastic connecting element (10) is formed by an elastic pad (101).

5. The artificial foot as claimed in any one of Claims 1 to 4, **characterized in that** the elastic connecting element (10) is provided with a limiting device (11) limiting the distance between supporting structure (3) and sole structure (2).

6. The artificial foot as claimed in Claim 5, **characterized in that** the limiting device is formed by a flexible band (11) which is tensible with increasing distance of the upper supporting structure (3) from the sole structure (2).

7. The artificial foot as claimed in Claim 6, **characterized in that** the flexible band (11) is guided with deflection by the pad (101) so that elastic displacement by material of the pad (101) is required to tense the band (11).

8. The artificial foot as claimed in any one of Claims 1 to 7, **characterized in that** the upper supporting structure (3) is formed from a rigid material.

9. The artificial foot as claimed in any one of Claims 1 to 8, **characterized in that** the coupling system (4) is arranged on the toe-side end of the upper supporting structure (3).

10. The artificial foot as claimed in any one of Claims 1 to 9, **characterized in that** the upper supporting structure (3) has an underside (9) which increases from the toe-side end relative to the sole structure (2) so that is has at the heel-side end a greater distance from the sole structure than in the region of the coupling system (4).

11. The artificial foot as claimed in any one of Claims 4 to 10, **characterized in that** the elastic pad (101) has a horizontal slot (12), in which the sole structure (2) is pushed.

12. The artificial foot as claimed in any one of Claims 5 to 11, **characterized in that** the band (11) is looped around the underside of the sole structure (2).

13. The artificial foot as claimed in Claim 12, **characterized in that** a lug (13) which can be looped around by the band (11) on its top side is arranged on the upper supporting structure (3).

14. The artificial foot as claimed in any one of Claims 1 to 13, **characterized in that** the underside (9) of the supporting structure (3) is designed in the heel area roof-like.

15. The artificial foot as claimed in Claim 14, **characterized in that** the elastic pad (101) with a roof-like top side rests on the roof-like underside (9) of the supporting structure (3).

16. The artificial foot as claimed in any one of Claims 1 to 15, **characterized in that** the elastic connecting element (10) has a control spring (102) with a heel-aligned arm (17).

17. The artificial foot as claimed in any one of Claims 1 to 16, **characterized in that** a toe-aligned arm (19) of a control spring (102) forms part of the sole structure (2), in which the toe-aligned arm (19) is connected to the front end of a sole spring (21) of the sole structure (2).

18. The artificial foot as claimed in Claim 17, **characterized in that** the connection of the toe-aligned arm (19) to the sole spring (21) occurs via an elastic damper (20).

19. The artificial foot as claimed in Claim 18, **characterized in that** flexible retaining straps (22, 23) limiting the distance between sole spring (21) and the toe-aligned arm (19) are arranged in a longitudinal direction on both sides of the elastic damper (20).

20. The artificial foot as claimed in any one of Claims 4 to 19, **characterized in that** the elastic pad (101) is arranged adjustably in a longitudinal direction.

21. The artificial foot as claimed in any one of Claims 1 to 20, **characterized in that** a lockable height-adjustment device (27) is provided in terms of power flow in series with the elastic connecting element (10), with which the distance between the sole structure (2) and the upper supporting structure (3) can be adjusted.

22. A method for controlling movement of an artificial foot depending on the movement of a lower leg part (14) of a patient, **characterized in that** the movement of the lower leg part (14) is introduced multiaxially flexibly by means of a supporting structure (3) in an elastic sole structure (2) at an introductory point in a middle region relative to the length of the foot such that at least when the patient is standing no change in distance between supporting structure (3) and sole structure (2) occurs at the introductory point, and **in that** in the heel area the movement of the elastic sole structure (2) is damped elastically in the direction of the supporting structure (3) and is limited in the direction away from the supporting structure (3).

23. The method as claimed in Claim 22, **characterized in that** a flexing action in the toe area is controlled by the elastic sole structure (2).

24. The method as claimed in Claim 22 or 23, **characterized in that** a lessening of the distance is permitted between supporting structure (3) and sole structure (2) at the introductory point by higher weight forces for heel-toe walking as the patient walks.

## Revendications

1. Pied prothétique avec un axe longitudinal qui s'étend depuis une zone de talon jusqu'à une zone d'orteils, une longueur, une largeur et une hauteur, une connexion à une partie de jambe (14), une structure de soutien supérieure (3) en direction du haut, une structure de semelle élastique (2) qui s'étend depuis la zone de talon jusque la zone d'orteils et un élément de liaison élastique agencé entre la structure de soutien supérieure (3) et la structure de semelle (2), dans lequel la structure de soutien supérieure (3) est reliée, approximativement au milieu du pied par référence à sa longueur, à la structure de semelle (2) au moyen d'un agencement d'accouplement (4) qui permet un mouvement de basculement relatif entre la structure de soutien (3) et la structure de semelle (2), dans lequel l'agencement d'accouplement (4) est réalisé de telle façon qu'il maintient constante la distance entre la structure de soutien (3) et la structure de semelle (2) dans le milieu de l'agencement d'accouplement (4) au moins lors d'une charge pondérale dans la situation debout du patient, et dans lequel la transmission des forces de la partie de jambes vers la structure de semelle a lieu grâce à cela à un emplacement d'application défini et restant sans modification approximativement au milieu du pied,
**caractérisé en ce que** la structure de soutien supérieure (3) est en un matériau dont l'élasticité est nettement plus faible que l'élasticité de la structure de semelle.

2. Pied prothétique selon la revendication 1, **caractérisé en ce que** l'agencement d'accouplement (4) est réalisé par une pièce de matière (5) qui est compressible par des couples de basculement sur une partie de sa section transversale.

3. Pied prothétique selon la revendication 1 ou 2, **caractérisé en ce que** l'agencement d'accouplement (4) est compressible sous des forces plus élevées, comme il s'en produit lors du processus de déambulation, pour la réduction de la distance au milieu de l'agencement d'accouplement (4).

4. Pied prothétique selon l'une des revendications 1 à 3, **caractérisé en ce que** l'élément de liaison élastique (10) est formé par un rembourrage élastique (101).

5. Pied prothétique selon l'une des revendications 1 à 4, **caractérisé en ce que** l'élément de liaison élastique (10) est pourvu d'un moyen de limitation qui limite la distance entre la structure de soutien (3) et la structure de semelle (2).

6. Pied prothétique selon la revendication 5, **caractérisé en ce que** le moyen de limitation est formé par une bande flexible (11) qui peut être mise sous tension en cas d'augmentation de la distance de la structure de soutien (3) depuis la structure de semelle (2).

7. Pied prothétique selon la revendication 6, **caractérisé en ce que** la bande flexible (11) est guidée avec un renvoi autour du rembourrage (101), de sorte que pour mettre la bande sous tension (11) il est nécessaire d'effectuer un refoulement élastique du matériau de rembourrage (101).

8. Pied prothétique selon l'une des revendications 1 à 7, **caractérisé en ce que** la structure de soutien supérieure (3) est formée en un matériau rigide.

9. Pied prothétique selon l'une des revendications 1 à 8, **caractérisé en ce que** l'agencement d'accouplement (4) est agencé à l'extrémité côté orteil de la structure de soutien supérieure (3).

10. Pied prothétique selon l'une des revendications 1 à 9, **caractérisé en ce que** la structure de soutien supérieure (3) comporte une face inférieure (9) qui monte depuis l'extrémité côté orteil par rapport à la structure de semelle (2), de sorte qu'elle présente à l'extrémité côté talon une distance plus élevée par rapport à la structure de semelle que dans la région de l'agencement d'accouplement (4).

11. Pied prothétique selon l'une des revendications 4 à 10, **caractérisé en ce que** le rembourrage élastique (101) comporte une fente horizontale (12) dans laquelle est enfilée la structure de semelle (2).

12. Pied prothétique selon l'une des revendications 5 à 11, **caractérisé en ce que** la bande (11) fait une boucle autour de la face inférieure de la structure de semelle (2).

13. Pied prothétique selon la revendication 12, **caractérisé en ce qu'**un prolongement (13) susceptible d'être entouré par la bande (11) à sa face supérieure est agencé sur la structure de soutien supérieure (3).

14. Pied prothétique selon l'une des revendications 1 à 13, **caractérisé en ce que** la face inférieure (9) de la structure de soutien (3) est réalisée en formant un toit dans la zone de talon.

15. Pied prothétique selon la revendication 14, **caractérisé en ce que** le rembourrage élastique (101) est appliqué avec une face supérieure en forme de toit contre la face inférieure en forme de toit (9) de la structure de soutien (3).

16. Pied prothétique selon l'une des revendications 1 à 15, **caractérisé en ce que** l'élément de liaison élastique (10) comporte un ressort de commande (102) avec un bras orienté vers le talon (17).

17. Pied prothétique selon l'une des revendications 1 à 16, **caractérisé en ce qu'**un bras (19) orienté vers les orteils, d'un ressort de commande (102) constitue une partie de la structure de semelle (2), dans laquelle le bras (19) orienté vers les orteils est relié à l'extrémité avant d'un ressort de semelle (21) de la structure de semelle (2).

18. Pied prothétique selon la revendication 17, **caractérisé en ce que** la liaison du bras (19) orienté vers les orteils avec le ressort de semelle (21) a lieu via un amortisseur élastique (20).

19. Pied prothétique selon la revendication 18, **caractérisé en ce que** des bandes de retenue flexibles (22, 23) sont agencées en direction longitudinale des deux côtés de l'amortisseur élastique (20), lesquelles limitent la distance entre le ressort de semelle (21) et le bras (19) orienté vers les orteils.

20. Pied prothétique selon l'une des revendications 4 à 19, **caractérisé en ce que** le rembourrage élastique (101) est agencé de manière déplaçable en direction longitudinale.

21. Pied prothétique selon l'une des revendications 1 à 20, **caractérisé en ce qu'**un dispositif de réglage en hauteur (27) verrouillable est prévu, pour ce qui concerne le flux des efforts, en série avec l'élément de liaison élastique (10), dispositif avec lequel la distance entre la structure de semelle (2) et la structure de soutien supérieure (3) est réglable.

22. Procédé pour la commande du déplacement d'un pied prothétique en fonction du mouvement d'une partie de jambe (14) d'un patient, **caractérisé en ce que** le mouvement de la partie de jambe (14) est appliqué, au moyen d'une structure de soutien (3) dans une structure de semelle élastique (2) à un emplacement d'application défini, dans une zone médiane, par référence à la longueur du pied, de manière articulée autour de plusieurs axes de telle façon qu'au moins lors de la station debout du patient il ne se produit aucune modification de la distance entre la structure de soutien (3) et la structure de semelle (2) à l'emplacement d'application, qui reste sans modification, et **en ce que**, dans la zone de talon, le mouvement de la structure de semelle élastique (2) en direction de la structure de soutien (3) est amorti de manière élastique et limité dans la direction en éloignement de la structure de soutien (3).

23. Procédé selon la revendication 22, **caractérisé en ce qu'**un mouvement de déroulement dans la zone des orteils est contrôlé par la structure de semelle élastique (2).

24. Procédé selon la revendication 22 ou 23, **caractérisé en ce que** pour un déroulement dans le processus de déambulation du patient, une diminution de la distance entre la structure de soutien (3) et la structure de semelle (2) au niveau de l'emplacement d'application sous des forces pondérales plus élevées est autorisée.
